# EUROPEAN PATENT APPLICATION

(11) **EP 1 304 117 A1**
(43) Date of publication of application: **23.04.2003**
(21) Application number: 01949921.9
(22) Date of filing: 10.07.2001
(51) Int. Cl.: A61K 38/17, A61P 19/00, A61P 19/10

(54) **REMEDIES FOR BONE DISEASES**

(30) Priority: 11.07.2000 JP 2000209926
(71) Applicant: BML, Inc., Tokyo 151-0051 (JP)
(72) Inventor: YOSHIKO, Yuji, c/o Department of Dentistry, Hirosshima-shi, Hiroshima 734-8553 (JP); KOIDE, Yoshio, Izumi-ku, Sendai-shi, Miyagi 981-3214 (JP); IGARASHI, Akira, c/o Laboratory, BML, INC., Kawagoe-shi, Saitama 350-1101 (JP); TAKANO, Shoichi, c/o Laboratory, BML, INC., Kawagoe-shi, Saitama 350-1101 (JP); MAEDA, Norihiko, c/o Department of Dentistry, Hiroshima-shi, Hiroshima 734-8553 (JP); AUBIN, Jane E.,c/o Faculty of Medicine, Toronto, Ontario M5S 1A8 (CA)
(74) Representative: Bohmann, Armin K., Dr.
(86) International application number: JP0105962
(87) International publication number: WO02004013

(57) **Abstract**

A therapeutic drugs for bone diseases containing as the active ingredient staniocalcin 1 which is found to have an effect of increasing bone mass. This drug for bone diseases is effective for bone diseases accompanied by anomalous osteogenesis or reduction in bone mass, such as osteoporosis, traumatic bone injuries, osteomalacia, rheumatic bone diseases, cancer-associated bone diseases, bone diseases associated with phosphorus metabolic error or calcium metabolic error, rachitis, and arthrosis deformans.

## Description

### Technical Field

The present invention relates to a therapeutic drug for bone diseases such as osteoporosis.

### Background Art

Most bone diseases are characterized by close participation, in one way or another, of reduction in bone mass per unit volume, and one typical form of pathological bone condition is osteoporosis.

Osteoporosis collectively refers to pathological conditions in which bone mass is anomalously reduced as a result of any of a variety of causes, and includes, among others, 1) senile osteoporosis and postmenopausal osteoporosis, 2) endocrine osteoporosis, 3) congenital osteoporosis, and 4) osteoporosis from immobilization and post-traumatic osteoporosis.

In particular, calcium intake has tended to decrease in recent years, because of the aging of society and the adoption of a western-style diet.

Thus, prevention and therapy of bone diseases involving the above-mentioned reduction in bone mass has become of increasing importance, and *inter alia*, development of effective therapeutic drugs for bone diseases has been awaited.

Existing therapeutic drugs for bone diseases include calcium-containing agents, vitamin-D-containing drugs, female hormone drugs, ipriflavone, and vitamin K2 drugs. However, none of these can be said to produce satisfactory results.

An object of the present invention is to identify a substance capable of effectively increasing bone mass, to thereby provide a therapeutic drug for bone disease, particularly osteoporosis or like pathological conditions in which reduction in bone mass is involved, the therapeutic drug containing the substance as an active ingredient.

### Disclosure of the Invention

The present inventors have performed extensive studies, and have found that staniocalcin 1 (hereinafter may be referred to as STC1) a glycoprotein which participates in calcium metabolism exhibits excellent osteogenesis-promoting effect, thereby leading to provision of a therapeutic drug for bone diseases.

Accordingly, the present invention provides a therapeutic drug for bone diseases (hereinafter may be referred to as the present therapeutic drug) containing, as an active ingredient, staniocalcin 1 (STC1). In the present invention, "therapeutic drug" is used in a broad sense, and encompasses not only drugs used for treatment in the narrow sense (i.e., treatment of a disease that presently affects a patient) but also preventive drugs.

With regard to technology related to the present invention, some findings regarding staniocalcin α (STCα) are disclosed in Japanese *Kohyo* (PCT) Patent Publication No. 509036/1998.

However, STCα (which is substantially identical with STC2) differs greatly from STC1 not only in amino acid sequence but also in function. Specifically, in contrast to STC1, STC2 (which is substantially identical with STCα) suppresses the promoter activity of NaPi-3, and prevents intake of phosphate into kidney cells (OK cells) (Ishibashi K. *et al.*, B.B.R.C., Res. 250, 252-258 (1998)).

Although the above patent publication refers to the effect of STCα on bone diseases such as osteoporosis, no specific data are provided therein. The effect mentioned therein is merely a prediction deduced from the analogy between parathyroid hormone (PTH) and STC in terms of function. Thus, as is evident, the above publication contains substantially no disclosure as to what effect STCα exerts on bone diseases.

As described above, Japanese *Kohyo* Patent Publication No. 509036/1998 fails to disclose or suggest a subject matter related to the present invention.

### Brief Description of the Drawings

Fig. 1 shows the nucleotide sequence of a staniocalcin 1 gene and an amino acid sequence corresponding thereto;
Fig. 2 shows staining images of calvarial cell culture samples in wells, which samples have been subjected to alkaline phosphatase staining or Von Kossa staining;
Fig. 3 shows the results of a study regarding the number of calvarial-cell-derived bone nodules confirmed when staniocalcin 1 was added in the absence of dexamethasone;
Fig. 4 shows the results of a study regarding the number of calvarial-cell-derived bone nodules confirmed when staniocalcin 1 was added in the presence of dexamethasone;
Fig. 5 shows the results of a comparative study regarding calvarial-cell-derived bone nodules confirmed when staniocalcin 1 was added in the presence and absence of dexamethasone; and
Fig. 6 shows results of a study regarding how addition of staniocalcin 1 affects expression of a marker of mature osteoblasts.

### Best Modes for Carrying Out the Invention

Modes of the present invention will next be described.

### A. Active ingredient of the present therapeutic drug

Preferably, STC1, the active ingredient of the present therapeutic drug, is an STC1 of human origin.

Staniocalcins (STCs) identified in the class of osteichthyes are glycoproteins secreted by the corpuscles of Stannius peculiar to the osteichthyes. They primarily act on Ca-ATPase contained in the gill and function to suppress the blood calcium level (Hirano T., Vertebrate Endocrinology: Academic Press, San Diego, vol. 3, 139-169, 1989; and Wagner G.F., Fish Physiol., 13, 273-306, 1994). At the time STC was discovered, STC was considered a hormone unique to the osteichthyes. However, in recent years, a human gene having a nucleotide sequence exhibiting high homology to STC originating from osteichthyes has been successfully cloned (Chang A.C.M., *et al.,* Mol. Cell Endocrinol., 112, 241-247, 1995; Olsen H.S., *et al.,* Proc. Natl. Acad. Sci. U.S.A., 93, 1792-1796, 1996; genebank NM003155; and genebank U46768), and subsequent studies have confirmed the presence of the substance in mouse (Chang A.C.M., *et al.,* Mol. Cell Endocrinol., 124 (1-2), 185-187, 1996 and genebank MMU47815), rat (genebank U62667), and dog (genebank AF178116), thereby substantiating that STC is ubiquitously present in mammals. Some time later, staniocalcin having an amino acid sequence different from that of the originally discovered STC was identified (Chang A.C.M., *et al.,* Mol. Cell Endocrinol., 141 (1-2), 95-99, 1998; Ishibashi K., *et al.,* Biochem. Biophys. Res. Commun., 250(2), 252-258, 1998; genebank AF055460; and genebank AB012664). Thus, the STC originally discovered was named STC1, and the second STC discovered was named STC2, and since then, STC1 and STC2 have been acknowledged to be two separate substances.

To date, the roles of STC1 have not been elucidated clearly. From the limited number of reports, STC1 is known to promote intake of phosphorus occurring in the kidney and the small intestine, and to inhibit intake of calcium in the small intestine (see, for example, Wagner G.F., *et al.*, Journal of Bone and Mineral Research, vol. 12, No. 2, pp 165-171, 1997; and Madsen K.L., *et al.,* Am. J. Physiol., 274 (1 pt 1), G96-102, 1998).

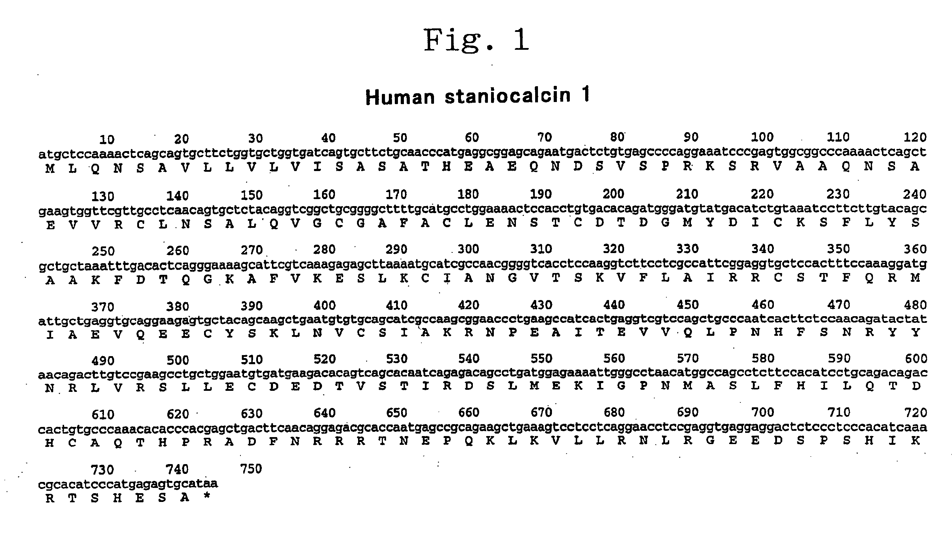

As described above, the amino acid sequence of STC1 protein and a gene coding for the protein have already been clarified. Fig. 1 shows these sequences. In Fig. 1, the amino acid residues described in lower rows corresponding to their upper-row nucleotide sequence are on the one-letter representation basis, where A: alanine, V: valine, L: leucine, I: isoleucine, P: proline, F: phenylalanine, W: tryptophan, M: methionine; G: glycine; S: serine; T: threonine; C: cysteine; Q: glutamine; N: asparagine; Y: tyrosine; K: lysine R: arginine; H: histidine; D: aspartic acid; and E: glutamic acid. As used herein, STC1, which serves as the active ingredient of the therapeutic drug of the present invention, encompasses, in addition to the STC1 of natural type having the above-described amino acid sequence, similar glycoproteins having partially modified amino acid sequences which may be obtained by modifying the natural type STC1 through a conventional method, for example, site-specific mutagenesis (Mark, D.F., et al., Proc. Natl. Acad. Sci. U.S.A., 81, 5662 (1984)), or fragments of the resultant peptides, so long as they exhibit biological activities that are considered substantially identical with those of natural STC1 (an acceptable amino acid sequence homology allows difference in amino acid sequence of 10% or thereabouts).

STC1 may be obtained by subjecting a biological material containing STC1 to extraction and purification procedures. Alternatively, in order to consistently mass-produce STC1, use of a recombinant obtained through a genetic engineering technique is more suitable and realistic.

STC1 can be prepared by use of a conventional method on the basis of a gene encoding STC1, which is already known as described above. For example, the following procedure may be performed: cDNA obtained from mRNA prepared from the kidney, the ovary, or similar material is employed as a template DNA, and also by use of a gene amplification primer designed on the basis of known STC1 nucleotide sequence, PCR or any other suitable gene amplification method is performed to thereby obtain a gene coding for the STC1 protein; alternatively, STC1 gene is obtained through a chemical synthesis method, such as phosphite-triester method, or by use of a DNA synthesizer making use of such a chemical synthesis method. The thus-obtained gene is inserted into a suitable gene expression vector; and the STC1 of interest can be obtained from a suitable host, such as *E. coli, Bacillus subtilis,* yeast, or insect cells, which has been transformed with such a recombinant vector.

Preferably, the above-mentioned gene expression vector possesses, among others, a promoter and an enhancer in the upstream region, and a transcription termination sequence in the downstream region.

Expression of the STC1 gene is not necessarily attained in a direct expression system. For example, there may be employed a fusion protein expression system which makes use of a β-galactosidase gene, a glutathione-S-transferase gene, or a thioredoxin gene.

Examples of a gene expression vector whose host is *E. coli* include pQE, pGEX, pT7-7, pMAL, pTrxFus, pET, and pNT26CII. Examples of a gene expression vector whose host is *Bacillus subtilis* include pPL608, pNC3, pSM23, and pKH80.

Examples of a gene expression vector whose host is yeast include pGT5, pDB248X, pART1, pREP1, YEp13, YRp7, and YCp50.

Examples of a gene expression vector whose host is a mammal cell or insect cell include p91023, pCDM8, pcDL-SRα296, pBCMGSNeo, pSV2dhfr, pSVdhfr, pAc373, pAcYM1, pRc/CMV, pREP4, and pcDNAI.

These gene expression vectors may be selected in accordance with the purpose of expression of STC1. For example, when STC1 is desired to be expressed in large amounts, a gene expression vector which allows use of *E. coli, Bacillus subtilis*, or yeast as a host is preferably employed. On the other hand, if ensured expression of STC1 though in small amounts is desired, a gene expression vector which allows use of a mammal cell or insect cell as a host is preferably employed.

Although a gene expression vector chosen from among existing ones may be selected as described above, as a matter of course, an appropriate gene expression vector may be created in accordance with the purpose of expression.

The aforementioned gene expression vectors in which the STC1 gene is inserted are transferred to host cells, and then the cells are transformed through a conventional method; for example, the calcium chloride method or electroporation in the case where the host is *E. coli* or *Bacillus subtilis*; or the calcium phosphate method, electroporation, or the liposome method in the case where the host cells are mammal cells or insect cells.

The resultant transformed cells are cultured through a conventional method, to thereby yield STC1 of interest.

In culturing, a culture medium is appropriately selected in accordance with the nature of the host. For example, when the host is *E. coli*, LB medium or TB medium may be used, and when the host is a mammal cell, RPMI1640 medium may be used.

The STC1 can be isolated and purified from the resultant culture product through a conventional method by, for example, subjecting the culture product to any of a variety of treatment procedures making use of physical and/or chemical properties of STC1.

Specifically, the isolation and purification procedure may make use of treatment with a protein precipitant, ultrafiltration, gel filtration, high-performance liquid chromatography, centrifugal separation, electrophoresis, affinity chromatography making use of a specific antibody, or dialysis. These may be employed singly or in combination.

In this way, STC1 can be isolated and purified.

STC1 promotes osteogenesis and thus is effective for the therapy and prevention of bone diseases, particularly osteoporosis or like pathological bone conditions accompanied by anomalous osteogenesis or reduction in bone mass. Specifically, STC1 is effective for the therapy and prevention of the mentioned osteoporosis [e.g., 1) senile osteoporosis and postmenopausal osteoporosis, 2) endocrine osteoporosis, 3) congenital osteoporosis, and 4) osteoporosis from immobilization and post-traumatic osteoporosis], osteomalacia, rheumatic bone diseases, cancer-associated bone diseases, traumatic bone injuries such as fracture, bone diseases associated with phosphorus metabolic error or calcium metabolic error, rachitis, and arthrosis deformans.

### B. Form of the present therapeutic drug

The present therapeutic drug contains STC1 as an active ingredient thereof, and may also contain, along with STC1, a suitable pharmaceutically acceptable carrier, to thereby yield a formulated drug product (needless to say, use of STC1 alone is possible). In accordance with the specific drug form of interest, the pharmaceutically acceptable carrier may be arbitrarily chosen from among diluents, excipients (such as a filler, a volume-increasing agent, a binder, a wetting agent, a stabilizer, a solubilizer, a disintegrant, and a surfactant), and other conventionally recognized pharmaceutically acceptable carriers. No particular limitations are imposed on the form of the drug composition, so long as it permits effective use of STC1 in the therapy of bone diseases such as osteoporosis. For example, the drug may have a solid form, examples of which include tablets, powder, granules, and pills; alternatively, the drug may have an injection form, examples of which include liquid, suspension, and emulsion. Further alternatively, STC1 may take a dry form which can be transformed to a solution upon addition of a suitable carrier before use.

No particular limitations are imposed on the dose of the thus-obtained therapeutic drug of the present invention, and the dose may be appropriately determined in accordance with the administration route of the drug, the form of the drug to be administered, the pathological condition of the patient, and other factors. Typically and preferably, a drug product containing the active ingredient STC1 in an amount of approximately 0.00001 to 90 by mass% is prepared, and administered to a patient once a day or several times a day, to thereby attain a daily STC1 dose of about 10 µg to about 10 mg for an adult.

Drug products having any of the above-mentioned forms may be administered to a patient via a suitable administration route in accordance with the form; in the case where the drug is prepared in an injection form, it can be administered, for example, intravenously, intramuscularly, endosteally, intraarticularly, subcutaneously, intradermally, or intraperitoneally; and in the case where the drug is prepared in a solid form, it can be administered, for example, orally, or enterally.

### Examples

The present invention will next be described in more detail by way of examples, which should not be construed as limiting the technical scope of the invention.

### [Test Example]

### (1) Materials and methods

### 1) Preparation of STC1

The STC1 subjected to the present Test Example is recombinant human staniocalsin 1 (r-hSTC1) obtained by use of *E. coli* as a host.

r-hSTC1 was prepared through a conventional method, the general procedure of which is described above. Briefly, RNA was obtained from a human kidney by use of Trizol (Gibco BRL). The entirety of the thus-obtained RNA, together with a primer origo dT, was applied to a Superscript II (product of Gibco BRL), to thereby prepare cDNA. For gene amplification through PCR, a GeneAmp PCR system 2400 (Perkin Elmer) was employed. The primers for the target gene were designed on the basis of the previously reported nucleotide sequence of the gene (genebank MMU47485) by use of MIT Center for Genome Research (WWW Primer Picking (primer 3)). A PCR amplification cycle consisting of the processes of thermal denaturation (94°C for 30 sec) → annealing (56°C for 30 sec) → elongation (72°C for 30 sec) was performed 35 times.

The thus-obtained STC1 gene was inserted into a gene expression vector (pQE-30, product of Quiagen) to be used with *E. coli.* Subsequently, a host *E. coli* (JM109) integrated with the STC1-gene-inserted vector was transformed. Through analysis of the sequences of the resultant transformants, a transformant capable of producing STC1 was selected.

Subsequently, the selected transformant was cultured in a TB medium, and expression of STC1 was induced with IPTG. The cells were ultrasonically lysed, to thereby yield a fraction containing STC1. The fraction was subjected to metal-ion affinity chromatography, whereby an aqueous r-hSTC1 solution (1 mg/mL) was obtained.

### 2) Preparation of drug formulation (for injection)

Gelatin hydrolysate (100 mg) and mannitol (200 mg), serving as stabilizers, were added to the aqueous r-hSTC1 solution prepared in step 1) above (1 mg/mL; 10 mL). Distilled water was added to the mixture so as to make the total volume 100 mL. The resultant solution was applied to a membrane filter (0.22 µm) for filtration, and sterilized. Aliquots of the sterilized solution were dispensed to vials (1 mL/vial) and then subjected to freeze-drying, to thereby yield aseptic formulations each containing 100 µg r-hSTC1 per vial.

In the following tests, the thus-obtained r-hSTC1 formulations were used (immediately before use, the formulations were diluted with phosphate buffered saline (PBS)). Throughout the following tests, the amount of r-hSTC1 formulation actually employed is recalculated in terms of r-hSTC1.

### 3) Culture

Calvariae (about 30 pieces) were removed from fetuses of Wistar rats (21 days of pregnancy). All the calvariae were combined and chopped into fragments, and the fragments were treated with collagenase repeatedly (for 10 to 20 minutes per treatment; 5 repetitions of treatment), to thereby obtain fractions containing calvarium-derived cells. Each of the thus-obtained fractions, excepting the first fraction, was preincubated in an αMEM supplemented with 10% fetal calf serum (FCS-αMEM) for 24 hours (conditions: 5% CO₂ at 37°C). Cell debris were removed by washing, and then the calvarium-derived cells from the respective fractions were combined, followed by regulation of the cell count by use of FCS-αMEM. The cells were then seeded onto the wells of a 24-well plate in an amount of 5,000 to 8,000 cells per well. Following the seeding, the cells were cultured (5% CO₂ at 37°C) for 24 hours, and the medium was exchanged for fresh FCS-αMEM containing either (i) only 28µM ascorbic acid or (ii) 28µM ascorbic acid and 10nM dexamethasone (DEX). When the cells had become confluent in the course of incubation (5% CO₂ at 37°C) , β-glycerophosphate (β-GP) was added to each well to a final concentration of 10 mM. Until the cells had become confluent (note: confluency was reached on day 6 following the exchange of the medium), r-hSTC1 was added to the well once every day. Several test groups were established in accordance with the predetermined stepwise concentrations ranging from 200 ng to 2 fg. Four to five wells were allotted to each concentration of r-hSTC1. The medium was replaced every two or three days.

### 4) Quantitation of calcification

On a day between the fourteenth and twenty-first days after the above-described secondary culture, ALP (alkaline phosphatase) positive, calcified bone nodules were histochemically detected. ALP staining, and Von Kossa staining for identifying calcification substrate were carried out as follows.

The culture product was washed with cold PBS, and then sequentially subjected to the following steps: fixing with cold 10% neutral buffered formalin for 15 minutes; washing with water; staining with ALP color-developer [naphthol AS MX 5 mg; N,N-dimethylformamide 200 µL, 0.2M Tris-HCl buffer (pH 7.4) 25 mL; purified water 25 mL; and First Violet LB 30 mg] for 40 minutes; washing with water; staining with 2.5% silver nitrate solution for 30 minutes; washing with water; fixing for 3 minutes with 5% sodium carbonate and 25% formalin; washing with water; and drying. The resultant sample was observed under a microscope for counting the number of bone nodules. The count was statistically processed by means of JMP, and subjected to the multiple comparison test.

### 5) Semi-quantitative analysis of the amount of gene expression of osteoblast differentiation marker through RT-PCR

On the fourteenth day and the twenty-first day after the secondary culture described above, contents of at least three wells were combined, and RNA was recovered in its entirety by use of Trizol (Gibco BRL). The entirety of the thus-obtained RNA (2µg), together with a primer origo dT, was applied to a Superscript II (product of Gibco BRL), to thereby prepare cDNA. For gene amplification through PCR, a GeneAmp PCR system 2400 (Perkin Elmer) was employed. The primers for the target gene were designed on the basis of the following gene nucleotide sequences, which have been reported previously [(i) ALP: genebank M61704, (ii) bone sialo protein (BSP): genebank L20232, (iii) osteocalcin (OCN): genebank L24429, (iv) STC1: genebank MMU47485, and (v) ribosomal enzyme L32 (internal standard): genebank M35397], by use of MIT Center for Genome Reserch (WWW Primer Picking (primer 3)). A unit cycle for amplification through the semi-quantitative PCR consists of the process of thermal denaturation (94°C for 30 sec) → annealing (56°C for 30 sec) → elongation (72°C for 30 sec), and the numbers of repetitions are as follows: (i) ALP: 21-26 cycles, (ii) BSP: 20-26 cycles, (iii) OCN: 20-26 cycles, (iv) STC1: 22-28 cycles, and (v) ribosomal enzyme L32 (internal standard): 17-21 cycles.

The respective genes were subjected to the above-described amplification cycles for amplification, and the resultant amplified products were electrophoresed on 2% agarose gel, then stained with ethidium bromide. Further, the respective amplification products were subcloned and their sequences were verified.

### (2) Results

### 1) Bone nodule count

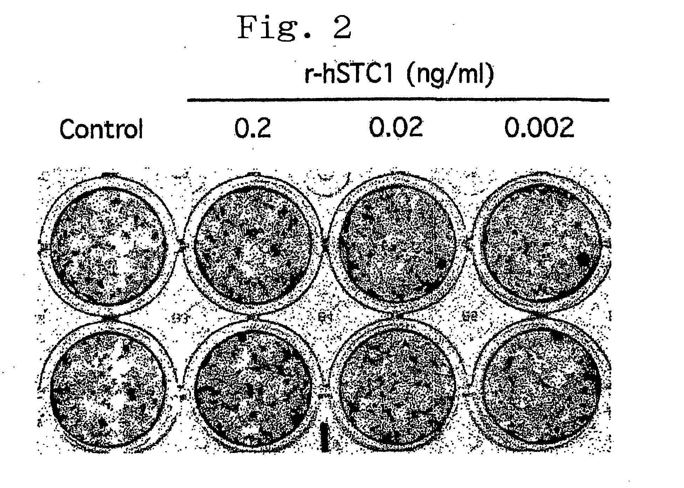

### a) Images of ALP staining and Von Kossa staining (Fig. 2)

Fig. 2 shows staining images obtained from culture products on day 14 of culture using a medium supplemented with DEX, β-GT, and ascorbic acid. The two leftmost wells (i.e., the upper left and lower left images) represent controls. In the subsequent pairs of wells, the amount of r-hSTC1 added to the media is changed stepwise towards the right, as follows: 0.2 ng/mL r-hSTC1, 0.02 ng/mL r-hSTC1, and 0.002 ng/mL. ALP stains red, and calcification substrate stains black. In Fig. 2, as compared with the control images, the remaining images show numerous black-colored portions indicating calcification caused by r-hSTC1.

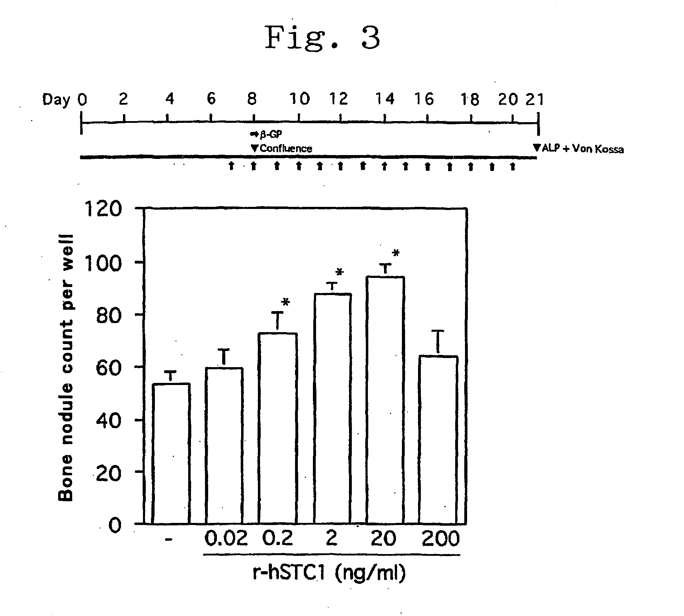

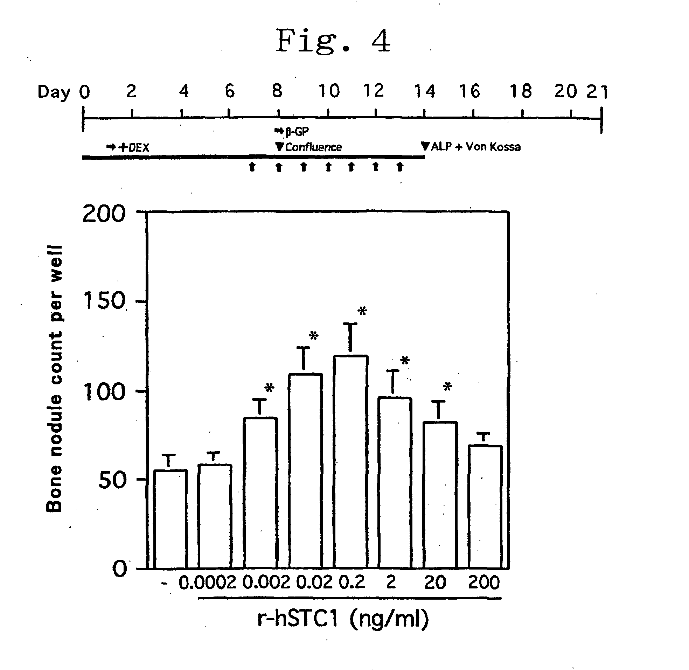

### b) Results on the 21st day of culture in the absence of DEX (Figs. 3 and 4)

As shown in Fig. 3, which corresponds to no addition of DEX, when r-hSTC1 was added, the bone nodule counts are significantly higher than the count identified in the control sample (p<0.05). Moreover, the maximum reaction of r-hSTC1 was found to be 20 ng/mL in the absence of DEX, whereas that of r-hSTC1 in the case where DEX was added was found to be 0.2 ng/mL (Figs. 3 and 4).

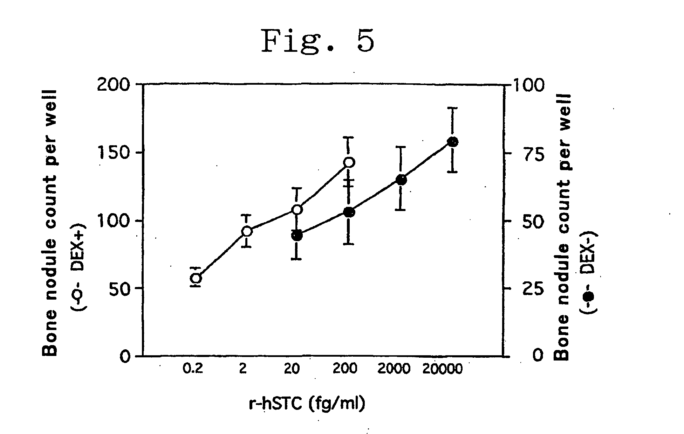

### c) Dose-response reaction of r-hSTC1 vs. nodule count (Fig. 5)

In Fig. 5, the plotting of the case where DEX was not added (DEX-) corresponds to day 21 of culture (on the basis of Fig. 3), and that of the case where DEX was added (DEX+) corresponds to day 14 of culture (on the basis of Fig. 4). In the presence of DEX, the maximum reaction shifted toward lower doses of r-hSTC1 (about 1/100).

### 2) Osteoblast marker

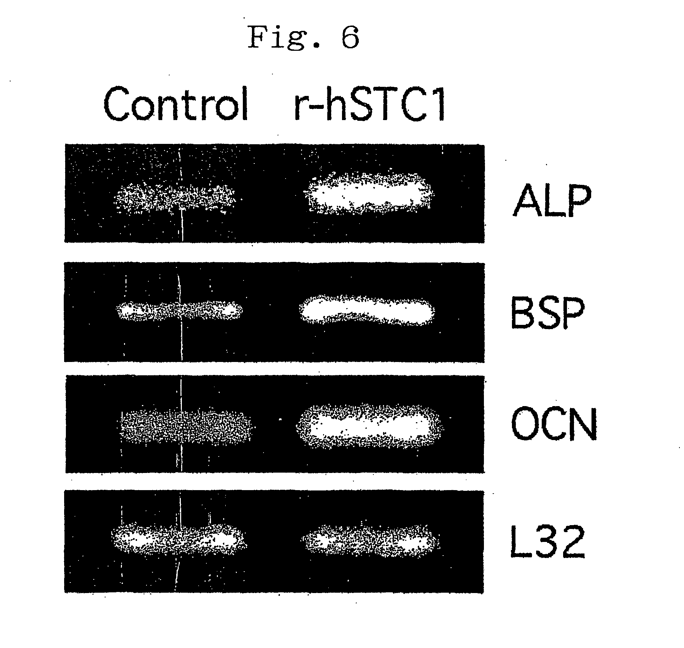

The results from item (1)-5) are shown in Fig. 6. As is apparent from Fig. 6, in the presence of r-hSTC1, the amount of expression of any of ALP, BSP, and OCN was found to be greater than that of control samples.

Taken together, the results confirm the following: 1) regardless of the absence or presence of DEX, formation of bone nodules is promoted by r-hSTC1; and 2) r-hSTC1 functions to increase the amount of expression of the genes of ALP, BSP, and OCN, which are mature osteoblast markers capable of forming bone nodules, thereby promoting osteogenesis.

As described above, the present invention has clarified that STC1 has a function of promoting osteogenesis, and has clearly substantiated that a therapeutic drug for bone diseases containing STC1 as the active ingredient thereof is effective for a variety of bone diseases, particularly such bone diseases accompanied by anomalous osteogenesis or reduction in bone mass, such as osteoporosis, traumatic bone injuries, osteomalacia, rheumatic bone diseases, cancer-associated bone diseases, bone diseases associated with phosphorus metabolic error or calcium metabolic error, rachitis, and arthrosis deformans.

### Industrial Applicability

The present invention provides a therapeutic drug for bone diseases involving reduced bone mass, such as osteoporosis.

## Claims

1. A therapeutic drug for a bone disease comprising staniocalcin 1 as an active ingredient thereof.

2. The therapeutic drug as recited in claim 1, wherein staniocalcin 1 is a human-derived staniocalcin 1.

3. The therapeutic drug as recited in claim 1 or 2, wherein the bone disease is a pathological condition accompanied by anomalous osteogenesis or reduction in bone mass.

4. The therapeutic drug as recited in claim 1 or 2, wherein the bone disease is selected from the group consisting of osteoporosis, traumatic bone injuries, osteomalacia, rheumatic bone diseases, cancer-associated bone diseases, bone diseases associated with phosphorus metabolic error or calcium metabolic error, rachitis, and arthrosis deformans.

5. The therapeutic drug as recited in claim 1 or 2, wherein the bone disease is osteoporosis.
